# EUROPEAN PATENT APPLICATION

(11) **EP 2 540 308 A1**
(43) Date of publication of application: **02.01.2013**
(21) Application number: 11747395.9
(22) Date of filing: 23.02.2011
(51) Int. Cl.: A61K 38/00, A61P 3/04, A61P 25/00, A61P 43/00

(54) **THERAPEUTIC AGENT FOR EATING DISORDERS**

(30) Priority: 24.02.2010 JP 2010039179
(71) Applicant: Morinaga Milk Industry Co., Ltd., Minato-ku Tokyo 108-8384 (JP)
(72) Inventor: SOMOTO,Yuuki, Zama-shi Kanagawa 252-8583 (JP); YAMADA,Akio, Zama-shi Kanagawa 252-8583 (JP); MATSUMOTO,Hiroshi, Zama-shi Kanagawa 252-8583 (JP)
(74) Representative: Albrecht, Thomas
(86) International application number: PCT/JP2011/054001
(87) International publication number: WO 2011/105435

(57) **Abstract**

Disclosed is a highly safe and effective therapeutic agent for eating disorders, which contains, as an active ingredient, a water-soluble fraction of a hydrolysis product of casein, said hydrolysis product being obtained by means of pepsin.

## Description

### TECHNICAL FIELD

The present invention relates to a therapeutic agent for eating disorders, which contains, as an active ingredient, a hydrolysis product of casein. Specifically, the present invention relates to a therapeutic agent for eating disorders, which contains, as an active ingredient, a hydrolysis product of casein having a molecular weight of 1 kDa to 25 kDa (kilodalton), which obtained by hydrolyzing a casein solution with pepsin.

### BACKGROUND ART

Eating disorders are diseases, which shows an extraordinary eating behavior. It is said that said disorders are caused by a mental factor(s). In order to distinguish the disorders from functional dysphagia and the like, recently, eating disorders are also called central eating disorders. Eating disorders are divided broadly into so-called anorexia (anorexia nervosa) and so-called hyperphagia (bulimia nervosa) and these make the transition to each other.

In anorexia, a patient loses his/her adequate appetite and therefore does not take foods sufficiently (refuses eating). The result of this behavior causes malnutrition, which sometimes leads to a serious condition(s) such as arrhythmia, anemia, amenorrhea, impaired liver function, osteoporosis. Treatments of these diseases resulted from the eating disorders are not ones which treat the eating disorders. While administering a nutritional supplement(s) forcibly is useful for treating the malnutrition, it is not a treatment for the eating disorders.

In hyperphagia, a patients cannot control his/her appetite due to bulimia, and thus, take foods too much (overeat). The result of this behavior sometimes leads to a serious condition(s) such as obesity, hyper-tension, diabetes. Treatments of these diseases resulted from the eating disorders are not ones which treat the eating disorders. While forced dietary regulation (dietetic therapy) is useful for preventing the excessive consumption (intake), it is not a treatment for the eating disorders. Since bulimia is not limited to a mealtime, dietary regulation and restriction of appetite are required from morning till night, which is not easy.

As a medicament for treating the eating disorders, for example, fluoxetine (product name: Prozac (registered trademark), Eli Lilly and Company, U.S., not approved in Japan) is available. However; fluoxetine is one of antidepressant (energizer) which is known as SSRI (selective serotonin reuptake inhibitor) which has a side effect on mental health, and therefore determining dosage thereof requires sufficient consideration, and thus, it is difficult to deal with the medicament.

Further, as a medicament for treating the eating disorders, especially hyperphagia, an appetite suppressant is sometimes used. As the appetite suppressant, only mazindol (product name: Sanorex (registered trademark), Novartis Pharma K.K.) is commercially available in Japan. However, mazindol is a psychotropic drug similar to amphetamine, and therefore dependence and resistance may be produced and it may have a side effect on mental condition, and thus, the medicament is not suitable for long term use and difficult to deal with. Further, suppression of appetite by mazindol and the like suppresses not only extraordinarily increased appetite but also appropriate appetite normally expressed, and therefore it has the problem as a medicament for treating the eating disorders.

As described above, all of the existing medicaments for treating the eating disorders (therapeutic agents for the eating disorders) have side effects on mental condition, and require sufficient considerations for determining the dosage, frequency of administration, dosing period and the like, and therefore it is difficult to deal with said medicaments, which cannot be used freely, and further cannot be used routinely (daily) for long term with no anxiety.

On the other hand, there are low calorie oral compositions added with polysaccharide and/or fibers, the object of which is to decrease the food intake by satisfying temporally the appetite at each meal by taking said composition before or while eating, instead of treating the eating disorders. Patent Document 1 describes a nutrition intervention composition for enhancing and extending satiety by taking the same before or while eating. However, said nutrition intervention composition described in Patent Document 1 is not practically effective for hyperphagia, since it is required to be taken before or while eating, although the extraordinarily increased appetite occurs at any time in addition to mealtime. Further, it is not practical to eat the low calorie composition for providing satiety, instead of meals, from morning till night.

Casein is a main milk protein ingredient separated from milk, which comprises four proteins encoded by different genes, namely, αs1 casein, αs2 casein, β casein and κ casein. It has been revealed that casein is incorporated by mammalians and exhibits several physiological effects. For example, Patent Document 2 describes a lipid-metabolism-improving agent containing αs-casein, as active ingredient. However, it has not yet been reported that decomposed (degraded) products of casein can be used as a therapeutic agent for the eating disorders.

### PRIOR ART DOCUMENTS

### PATENT DOCUMENTS

PATENT DOCUMENT 1: Japanese National Publication (Published Japanese Translation of PCT International Publication for Patent Application) No. 2003-523368
PATENT DOCUMENT 2: PCT International Publication No. WO2007/142230 pamphlet

### OUTLINE OF INVENTION

### PROBLEM TO BE SOLVED BY THE INVENTION

As described above, various food and drink and medicaments, and dietetic therapy using them have been studied for treating the eating disorders. However, said prior art has not necessarily achieved adequate effect. Therefore, more safe and effective agents for treating the eating disorders have been demanded.

Especially, the patients of hyperphagia cannot control their extraordinarily increased appetite by their will. Therefore, an agent for treating the eating disorders, without side effect on the patient's mental health, has been strongly required. In addition, an agent which suppresses only extraordinarily increased appetite and keeps the ordinary appetite expressed normally has been required.

Therefore, the object of the present invention is to provide a highly safe and effective agent for treating the eating disorders, preferably an agent for treating hyperphagia.

### MEANS FOR SOLVING PROBLEM

The present inventors had studied eagerly an active ingredient(s) for improving the eating disorders. Then, we found that a particular fraction of casein hydrolysate, among milk proteins, remarkably suppresses the extraordinarily increased appetite due to bulimia, and thus, treats the eating disorders, and the present invention has been completed.
The present invention provides an agent, containing a particular fraction of casein hydrolysate, for treating the eating disorders, preferably an agent for treating hyperphagia.
In preferred embodiment, the therapeutic agent according to the present invention includes, as an active ingredient, a water-soluble fraction of casein hydrolysate by pepsin.
In preferred embodiment, the therapeutic agent according to the present invention includes, as an active ingredient, casein hydrolysate having a molecular weight of 1 kDa to 25 kDa obtained by hydrolyzing casein with pepsin.
The therapeutic agent according to the present invention is preferably a casein hydrolysate which is obtained by hydrolyzing 1 mass part of casein with 1/10,000 to 1/1,000 mass part of pepsin.
In preferred embodiment, the therapeutic agent according to the present invention is preferably an agent which decreases the ingestion of meal by the patient of the eating disorders at least 10% (10% or more) after 5 weeks from the starting of administration.
In preferred embodiment, the therapeutic agent according to the present invention preferably treats the eating disorders, especially hyperphagia by suppressing an extraordinarily increased appetite (suppressing bulimia).

Accordingly, the present inventions comprise in the following (1) to (12).
(1)
   A therapeutic agent for eating disorders, comprises, as an active ingredient, a water-soluble fraction of casein hydrolysate by pepsin.
(2)
   The therapeutic agent for eating disorders according to (1), wherein the water-soluble fraction of casein hydrolysate by pepsin is the fraction which comprises, as an active ingredient, peptide fragment of a molecular weight of 1 kDa to 25 kDa.
(3)
   The therapeutic agent for eating disorders according to (1), wherein the water-soluble fraction of casein hydrolysate by pepsin comprises, as an active ingredient, peptide fragment of a molecular weight of 1 kDa to 20 kDa.
(4)
   The therapeutic agent for eating disorders according to (1), wherein the water-soluble fraction of casein hydrolysate by pepsin is the fraction which comprises at least 50% (50% or more) of peptide fragment of a molecular weight of 1 kDa to 20 kDa.
(5)
   The therapeutic agent for eating disorders according to (1), wherein the water-soluble fraction of casein hydrolysate by pepsin is the fraction which comprises, as an active ingredient, peptide fragment of a molecular weight of 1 kDa to 25 kDa, which is a hydrolysate of αs2-casein.
(6)
   The therapeutic agent for eating disorders according to (1), wherein the water-soluble fraction of casein hydrolysate by pepsin is the fraction which comprises, as an active ingredient, peptide fragment of a molecular weight of 1 kDa to 20 kDa, which is a hydrolysate of αs2-casein.
(7)
   The therapeutic agent for eating disorders according to any of (1) to (6), wherein a casein hydrolysate by pepsin is the hydrolysate obtained by hydrolyzing 1 mass part of casein with 1/10,000 to 1/1,000 mass part of pepsin.
(8)
   The therapeutic agent for eating disorders according to any of (1) to (7), wherein the water-soluble fraction is a supernatant fraction obtained by fractionating the casein hydrolysate by pepsin by decantation or centrifugation.
(9)
   The therapeutic agent for eating disorders according to any of (1) to (7), wherein the water-soluble fraction is a supernatant fraction obtained by centrifugation of casein hydrolysate by pepsin at 300 to 100000 G.
(10)
   The therapeutic agent for eating disorders according to any of (1) to (9), wherein said therapeutic agent for eating disorders is a long acting therapeutic agent for eating disorders.
(11)
   The therapeutic agent for eating disorders according to any of (1) to (10), wherein the eating disorder is hyperphagia.
(12)
   The therapeutic agent for eating disorders according to any of (1) to (11), wherein the ingestion per day in hyperphagia is decreased at least 10% after 5 weeks from the starting of administration, in comparison to the starting of administration.

As described above, the present invention comprises a therapeutic agent (medicament) for eating disorders, central eating disorders, anorexia, anorexia nervosa, hyperphagia, and bulimia nervosa, and also comprises a pharmaceutical agent (pharmaceutical composition) for eating disorders, central eating disorders, anorexia , anorexia nervosa, hyperphagia, and bulimia nervosa, and also comprises said therapeutic agent (medicament) and pharmaceutical agent (pharmaceutical composition) for long term use, and also comprises said therapeutic agent and pharmaceutical agent for oral administration. Said therapy includes treatment and prophylaxis (prevention), and further, also includes amelioration, improvement and remission. In preferred embodiment, the therapeutic agent (medicament) and pharmaceutical agent (pharmaceutical composition) according to the present invention can also be used as an agent for reducing the blood glucose level and a pharmaceutical agent for reducing the blood glucose level. In preferred embodiment, the therapeutic agent (medicament) and pharmaceutical agent (pharmaceutical composition) according to the present invention is applied to the eating disorders, preferably hyperphagia, and more preferably, for suppressing an excessively increased appetite (suppressing the excessively increased appetite), and the agent also comprises a suppressant for hyperphagia and a suppressant for the excessively increased appetite. In preferred embodiment, by the therapeutic agent (medicament) and pharmaceutical agent (pharmaceutical composition) according to the present invention, the suppression of hyperphagia preferably reduces the blood glucose level in the patient of hyperphagia.

Further; the present inventions also comprise the following (13) to (20).
(13)
   A method for preparing a therapeutic agent for eating disorders comprising steps of: obtaining casein hydrolysate by pepsin by hydrolyzing casein with pepsin, obtaining a water-soluble fraction from the casein hydrolysate by pepsin.
(14)
   The method according to (13) wherein
   the step of obtaining the casein hydrolysate by pepsin by hydrolyzing casein with pepsin is
   the step of obtaining the casein hydrolysate by pepsin by hydrolyzing casein with pepsin by adding 1/10000 to 1/1000 mass parts of pepsin to 1 mass part of casein.
(15)
   The method according to any of (13) to (14) wherein
   the step of obtaining a water-soluble fraction from the casein hydrolysate by pepsin is
   the step of obtaining a supernatant fraction as the water-soluble fraction by fractionating the casein hydrolysate by pepsin by decantation or centrifugation.
(16)
   The method according to any of (13) to (15) wherein
   the step of obtaining a water-soluble fraction from the casein hydrolysate by pepsin is
   the step of obtaining a supernatant fraction as the water-soluble fraction by fractionating the casein hydrolysate by pepsin by centrifugation of the hydrolysate at 300 to 100000 G.
(17)
   The method according to any of (13) to (16) wherein
   the therapeutic agent for eating disorders is a long acting therapeutic agent for eating disorders.
(18)
   The method according to any of (13) to (17) wherein
   the eating disorder is hyperphagia.
(19)
   The method according to any of (13) to (18), wherein the therapeutic agent for eating disorders is
   the therapeutic agent for eating disorders which decreases the ingestion of meal per day in hyperphagia at least 10% (10% or more) after 5 weeks from the starting of administration, in comparison to the starting of administration.
(20)
   The therapeutic agent for eating disorders which contains as an active ingredient, the water-soluble fraction obtained from the casein hydrolysate by pepsin, prepared by the method according to any of (13) to (19),.

Further, the present inventions also comprise the following (21) to (38).
(21)
   A method for treating the eating disorders by administrating the therapeutic agent for eating disorders according to any of (1) to (12).
(22)
   A method for treating the eating disorders of mammalian by administrating the therapeutic agent for eating disorders according to any of (1) to (12).
(23)
   The method according to any of (21) to (22), wherein
   the eating disorder is hyperphagia.
(24)
   The method according to any of (21) to (23), wherein
   the administration is an oral administration.
(25)
   The method according to any of (21) to (24), wherein
   the ingestion of meal per day in hyperphagia is decreased at least 10% after 5 weeks from the starting of administration, in comparison to the starting of administration.
(26)
   Use of the water-soluble fraction obtained from the casein hydrolysate by pepsin, in the manufacture of the therapeutic agent for eating disorders according to (1) to (12).
(27)
   The use according to (26), wherein
   the eating disorder is hyperphagia.
(28)
   A water-soluble fraction obtained from the casein hydrolysate by pepsin, for use in treating eating disorders.
(29)
   The water-soluble fraction according to (28), wherein
   the water-soluble fraction obtained from the casein hydrolysate by pepsin is the fraction which includes, as an active ingredient, peptide fragment of a molecular weight of 1 kDa to 25 kDa.
(30)
   The water-soluble fraction according to (28), wherein
   the water-soluble fraction obtained from the casein hydrolysate by pepsin includes as an active ingredient, peptide fragment of a molecular weight of 1 kDa to 20 kDa.
(31)
   The water-soluble fraction according to (28), wherein
   the water-soluble fraction obtained from the casein hydrolysate by pepsin contains at least 50% of peptide fragment of a molecular weight of 1 kDa to 20 kDa.
(32)
   The water-soluble fraction according to (28), wherein
   the water-soluble fraction obtained from the casein hydrolysate by pepsin is the fraction which contains as an active ingredient, peptide fragment of a molecular weight of 1 kDa to 25 kDa, which is a hydrolysate of αs2-casein.
(33)
   The water-soluble fraction according to (28), wherein
   the water-soluble fraction obtained from the casein hydrolysate by pepsin is the fraction which contains as an active ingredient, peptide fragment of a molecular weight of 1 kDa to 20 kDa, which is a hydrolysate of αs2-casein.
(34)
   The water-soluble fraction according to any of (28) to (34), wherein
   the casein hydrolysate by pepsin is obtained by hydrolyzing casein with pepsin by adding 1/10000 to 1/1000 mass parts of pepsin to 1 mass part of casein.
(35)
   The water-soluble fraction according to any of (28) to (34), wherein
   the water-soluble fraction obtained from the casein hydrolysate by pepsin is a supernatant fraction obtained by fractionating the casein hydrolysate by pepsin by decantation or centrifugation.
(36)
   The water-soluble fraction according to any of (28) to (34), wherein
   the water-soluble fraction obtained from the casein hydrolysate by pepsin is a supernatant fraction obtained by centrifugation of casein hydrolysate by pepsin at 300 to 100000 G.
(37)
   The water-soluble fraction of casein hydrolysate by pepsin according to any of (28) to (36), wherein
   the therapy of the eating disorders is a therapy of a long acting (prolonged) eating disorders.
(38)
   The water-soluble fraction of casein hydrolysate by pepsin according to any of (28) to
(36), wherein
   the therapy of the eating disorders is a therapy of hyperphagia.

According to the present invention, a highly safe and effective agent for treating the eating disorders can be obtained.
The therapeutic agent for the eating disorders according to the present invention is especially effective as a therapeutic agent for hyperphagia.
According to the present invention, the symptom of hyperphagia can be safely improved, since the present invention naturally suppresses only excessive appetite of the patient of hyperphagia and, on the other hand, does not suppress the normal appetite (the ordinary appetite).
The therapeutic agent for the eating disorders according to the present invention has a high safety for a human being and animals, and thus, can be taken routinely (daily) for long term without any anxiety. Accordingly, it is useful for treatment and prophylaxis (prevention) of various diseases occurred by the eating disorders secondary.
The casein hydrolysate is an active ingredient in the present invention and can be produced constantly in large scale from the relatively low cost materials such as milk, and therefore can be provided at a low price. Further, the therapeutic agent of the eating disorders also can be provided in the form of the food and drink, and feed (livestock feed).
The therapeutic agent for the eating disorders according to the present invention does not suppress the amount of meal ingestion by a feeling of fullness (plenitude), and therefore, the time of ingestion is not limited before and/or after the meal, and thus, it can be taken freely. Further, it is not necessary to be taken (ingested) every day because the effect thereof remians for a certain period (days).
The therapeutic agent for the eating disorders according to the present invention is one which naturally suppresses the excessive appetite merely by taking (ingesting) it, and thus, there is little stress to the patient's mental health, which is a problem for an ordinary dietary therapy.

### BRIEF DESCRIPTION OF DRAWINGS

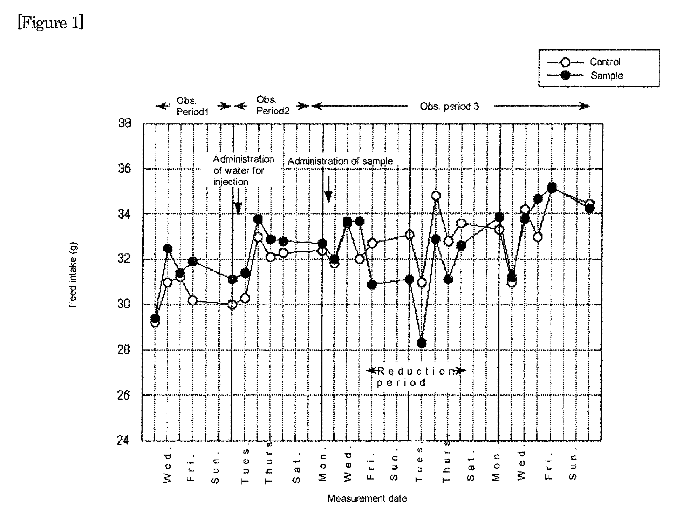

[Figure 1] Figure 1 is a graph indicating the change of the feeding pattern of ZDF rat.

### MODES FOR CARRYING OUT THE INVENTION

The preferred embodiments of the present invention is explained below. However, the present invention is not limited within the following preferred embodiments, and it can be modified freely within the scope of the present invention. In the present specification, percentage is expressed by mass (weight) unless otherwise indicated.

The therapy of hyperphagia by the present invention is one which suppresses only excessive appetite of the patient of hyperphagia and, on the other hand, maintains the normal appetite (the ordinary appetite). By the action of the agent for suppressing the hyperphagia according to the present invention, the amount of meal ingestion is improved into the appropriate range and the calorie intake also falls into the normal range. As the result, various symptoms which occur with the excessive amount of meal ingestion can be improved, for example, the effect of reduction of high value of blood glucose can be obtained. Accordingly, the therapeutic agent for hyperphagia according to the present invention is preferably used as an agent for reducing the blood glucose level thereof (blood glucose level reducing agent) for the patient of hyperphagia. Further, a method of suppressing the hyperphagia of mammals, and a method for reducing the blood glucose level of mammals with hyperphagia by administering the therapeutic agent for hyperphagia according to the present invention are provided.

The present invention also comprises a method for treating the eating disorders, preferably, a method for treating the eating disorders of mammalians. In "mammalians", human being and domestic animals (for example, horse, dog, cat, rabbit, cow, sheep, goat and the like) are included.

Further, as shown in the below-mentioned experimental examples, the therapeutic agent according to the present invention suppresses the excessive appetite characteristic of the hyperphagia but does not suppress the normal appetite (ordinary appetite). Therefore, there is no anxiety about health by suppressing the normal appetite. Accordingly, it can be used with no anxiety even if there is an anxiety that the hyperphagia and anorexia make the transition to each other. Therefore, the therapeutic agent according to the present invention is preferable as the therapeutic agent of hyperphagia and further preferable as the therapeutic agent of the eating disorders.

In the present invention, "treatment" (medical treatment) includes the effect which relieves (improves) the symptom and the effect of treating the disease. The effect obtained from the present invention is preferably the effect of inducing remission and the effect of maintaining the condition. When the therapeutic agent according to the present invention is routinely (daily) administered or taken, the therapeutic effects against the eating disorders, especially hyperphagia are exerted with little side effect.

### [Casein]

The starting material for the casein hydrolysate for the present invention is casein of milk protein. Casein, whose main ingredient is casein derived from the milk of mammalians (for example, cow, sheep, goat), can be used, and especially, casein derived from cow's milk can be preferably used. Any casein which can produce hydrolysate derived from αs2-casein can be used. For example, various caseins and caseinate commercially available, such as casein lactate, casein sulfate, casein hydrochloride, sodium caseinate, potassium caseinate, calcium caseinate, magnesium caseinate or mixture thereof and the like are preferable. Further, casein obtained from cow's milk, defatted milk, whole milk powder and non-fat dry milk (skim milk) by purification using a standard method can be used as a raw material. In a preferred embodiment, hydrolysis of casein is performed by adding the enzyme and the like into a casein solution prepared by dissolving casein. Therefore, in a preferred embodiment, an aqueous solution of casein can be preferably used as casein for the present invention.

### [Casein hydrolysate]

The casein hydrolysate for the present invention can be prepared as described below.
In the method for preparing the casein hydrolysate, firstly, casein is dispersed and dissolved in water, and thus, a casein solution is prepared. The concentration of casein in the casein solution is not particularly restricted (restrained). However, usually, 5 to 15 % by mass, as converted into protein mass (weight), is desirable in terms of efficiency and operability.

As for pH of the casein solution, it is preferable to adjust around the optimal pH for the protease used in the post-process. Since pepsin is used as the enzyme in the present invention, it is preferable to adjust the pH of the solution of raw material to the range of pH in which proteolysis by pepsin is possible, and thus, it is particularly preferable to adjust the pH in the range of 2 to 4. Adjusting the pH of the casein solution can be performed using a standard method. For example, it can be performed by adding an acidic solution. As the acidic solution used for adjusting the pH, it is preferable to use hydrochloric acid, sulfuric acid, nitric acid or the concentrate thereof and the like. These can be used alone, or can be used in combination with at least 2 of them.

In a preferred embodiment, it is preferable to sterilize the casein solution by heating and maintaining (incubating, keeping). It is preferable to sterilize the casein solution by heating after pH was adjusted. As for the condition of sterilization, for example, it can be performed at the temperature range of 80 to 120°C, preferably 85 to 100°C, for example, for the time in the range of 20 minutes to 1 second, preferably, in the range of 10 minutes to 10 seconds. After sterilization, the solution of raw material is preferably cooled to or no more than 42°C.

Subsequently, protease is added into the casein solution for hydrolyzing the casein by the enzymatic reaction, and thus, the solution of the hydrolysate of the casein is obtained. For obtaining the hydrolysate according to the present invention, it is most preferable to use pepsin as protease. As the pepsin for the present invention, for example, a commercially available pepsin can be used, pepsin sold by SIGMA or BIOPHEDEX being able to be exemplified. It is desirable to dissolve the enzyme in water and then add the solution.

The amount of pepsin to be added is important for determining the degree of hydrolysis of casein. As for the pepsin for the present invention, it is necessary to add 1/50,000 to 1/1,000 parts by mass of pepsin per 1 part by mass of casein. Preferably, 1/10,000 to 1/2,000 parts by mass of pepsin is added per 1 part by mass of casein. More preferably, 1/10,000 to 1/5,000 parts by mass of pepsin is added per 1 part by mass of casein. When the amount of pepsin added is too much, the hydrolysate which achieves the effect of the present invention cannot be obtained because proteolysis excessively proceeds. When the amount of pepsin added is too small , the hydrolysate which achieves the effect of the present invention cannot be obtained because proteolysis does not proceed.

The temperature during the enzymatic reaction is not particularly restricted. It is selected from the range which includes the optimal temperature range in which the enzyme action is expressed. Usually, the temperature for the enzymatic reaction is preferably 30 to 60°C. The degree of hydrolysis is varied on the basis of the reaction temperature. For example, the time (incubation time) for enzymatic reaction is preferably from 30 minutes to 16 hours when the temperature is 40 to 45°C. More preferably, the time is from 30 minutes to 4 hours. The time about 2 hours is most preferable.

In a preferred embodiment, the degree of hydrolysis of the casein hydrolysate obtained in the present invention is preferably 10 to 50%, more preferably 15 to 30%.
The degree of hydrolysis is calculated using the following formula.
The degree of hydrolysis (%) = (nitrogen in formol form / total nitrogen) x 100
wherein, the amount of total nitrogen in sample is determined by Kjeldahl method ("Shokuhin-Bunsekihou", Japanese Society for Food Science and Technology, p. 102, Korin Publishing Co., Ltd., 1984) and the amount of nitrogen in formol form in sample is determined by Formol titration ("Experiments in food science and technology", the first volume book, edited by Mitsuda et al, p. 547, Yokendo Co., Ltd., 1970).

It is preferable to perform the treatment of inactivating the enzyme after the treatment of hydrolyzing casein. As the treatment for inactivating the pepsin, it is preferable to perform the heat treatment for inactivating the pepsin. The temperature and time of the heat treatment for inactivating are set so that pepsin may be sufficiently inactivated. For example, the treatment can be performed at 85 to 120°C, preferably 85 to 100°C, for the retention time from 20 minutes to 10 seconds, preferably from 10 minutes to 10 seconds.

The treated solution (fluid) of casein hydrolysate thus obtained is usually a suspension containing the precipitate which includes a water-soluble fraction (supernatant fraction, soluble fraction) and insoluble fraction (precipitate fraction). Subsequently, from this treated solution of casein hydrolysate, precipitate is removed. As the method for removing the precipitate, various methods can be used. In a preferred embodiment, for example, centrifugation and micro filtration (MF filtration) can be used. Preferably, centrifugation can be used.

Centrifugation is performed using a centrifuge. As for the condition for the centrifugation, generally, it can be performed at 20,000 to 1,500 rpm, preferably, 20,000 to 3,000 rpm, more preferably, 10,000 to 3,000 rpm as rotating speed, and generally, 5 to 120 minutes, preferably 20 to 90 minutes, and more preferably, 20 to 60 minutes as rotating time (duration). For example, it can be performed at 10,000 to 3,000 rpm, for 20 to 60 minutes. However, it is not limited to said conditions. Further, generally, it can be performed at 300 to 100,000 G, preferably, 2000 to 100,000 G, and more preferably, 2000 to 50,000 G. For example, while it can be performed at 2000 to 50,000 G, for 20 to 60 minutes, it is not limited to said conditions. In a preferred embodiment, generally, it can be performed at 10,000 to 30,000 G, preferably, 15,000 to 25,000 G, more preferably, 18,000 to 22,000 G, and more preferably, 19,000 to 21,000 G. For example, while the centrifugation can be performed at these G, for 20 to 60 minutes, it is not limited to said conditions. After centrifugation, precipitate is removed, and the casein hydrolysate (water-soluble fraction of casein hydrolysate) according to the present invention can be obtained as a supernatant fraction (water-soluble fraction) from the centrifugation. It is also possible to powderize the casein hydrolysate according to the present invention by drying. As for the method for drying, hot-air drying or freeze-dry may be used.

The casein hydrolysate thus obtained according to the present invention included, as described below, as main ingredient, the hydrolysate derived from αs2-casein. The molecular weight of the casein hydrolysate according to the present invention is preferably within the range of no more than 37 kDa, more preferably within the range of 1 kDa to 37 kDa, and further more preferably, within the range of 1 kDa to 25 kDa, and further more preferably, 1 kDa to 20 kDa. The casein hydrolysate according to the present invention can be also deemed to be a composition comprising the soluble fraction (water-soluble fraction) of molecular weight of 1 kDa to 25 kDa obtained by hydrolyzing the casein solution by pepsin. It also can be deemed to be a composition comprising the soluble fraction (water-soluble fraction) of molecular weight of 1 kDa to 20 kDa obtained by hydrolyzing the casein solution by pepsin.

### [Therapeutic agent for eating disorders]

The therapeutic agent for eating disorders according to the present invention is particularly effective for hyperphagia, and thus, it can be used as a therapeutic agent for hyperphagia. The therapeutic agent according to the present invention can treat the hyperphagia by returning the amount of meal ingestion into the normal range by suppressing the excessive appetite of the patient with hyperphagia. The objective person to whom the therapeutic agent for eating disorders according to the present invention is administered is a patient with eating disorders, preferably a patient with hyperphagia, and more preferably, a patient with hyperphagia with anxiety of transition to anorexia.

While the therapeutic agent for eating disorders according to the present invention exerts the effect by only one time ingestion, from the time of the ingestion to the time when the effect is exerted, preferably, one to several days, more preferably, 1 to 6 days, further more preferably, 1 to 4 days, further more preferably, 1 to 3 days, further more preferably, 1 to 2 days, and preferably one day, preferably, 2 to 3 days, more preferably, 2 days are required. On the other hand, the effect exerted continues for about one week, preferably, 6 to 10 days, preferably, 7 to 9 days. The fact that the effect is exerted, instead of on the ingestion day, one to several days after the ingestion day and continues for about one week indicates that the therapeutic agent according to the present invention acts in the different mechanism from that of the low calorie compositions by prior art. The low calorie compositions by prior art decrease the amount of meal ingestion by satisfying temporally the appetite during the metal time by generating or promoting a feeling of fullness earlier by ingestion of the composition(s) before or while eating. In this point, the therapeutic agent according to the present invention is different from the agents for suppressing the appetite in prior art which exert the effect by generating or promoting a feeling of fullness.

A method for administering the therapeutic agent according to the present invention may be an oral administration, or parenteral administration such as enteral administration, and the method is not limited to them. In a preferred embodiment, it is preferable that the therapeutic agent according to the present invention is orally administered. In the case where the therapeutic agent is administered orally, it can be taken before or after the meal time, or between the meal times, and further; it can be taken in addition to the meal independently.

The active ingredient of the therapeutic agent according to the present invention is derived from, as the starting material, casein derived from milk. Milk, for example, ones obtained from cow, sheep, goat and the like has been used for drinking by human being for many years in our history, and therefore very high level safety for human being is ensured. Accordingly, the active ingredient of the therapeutic agent according to the present invention can be taken routinely (daily) and continuously for long term with no anxiety. Namely, it is different from the existing medicaments for treating eating disorders (therapeutic agent for eating disorders), and thus, has no mental side effect on the patient, and therefore, there is no strict restriction with regard to the dosage, frequency of administration, dosing period and the like, and thus, it is easy to deal with the therapeutic agent according to the present invention, and it can be used routinely (daily) and continuously for long term with no anxiety.

The therapeutic agent according to the present invention may be an agent comprising the casein hydrolysate according to the present invention alone, or an agent comprising the composition comprising the hydrolysate according to the present invention and other ingredient(s). In each case, the agent is preferably prepared as a preparation in dosage form suitable for use application.

The dosage form of the therapeutic agent according to the present invention is not particularly restrained (restricted). For example, it can be prepared in a known dosage form for oral administration such as a tablet, capsule, troche, sirup, granule, powder, emulsion, spray and the like. Alternatively, it is also possible to prepare a dosage form for parenteral administration such as a suppository, injectable solution, ointment, tape form and the like.

The dosage of the casein hydrolysate according to the present invention, which is an active ingredient, is varied by dosage form, symptom, age, body weight and the like. The dosage is preferably 83 mg/kg body weight per day or more, more preferably, 100 mg/kg body weight per day or more, more preferably, 120 mg/kg body weight per day or more for effectively exerting the effect of suppressing the hyperphagia. The casein hydrolysate according to the present invention has a high level safety, and therefore upper-limit of the dosage thereof is not restrained (restricted). However, especially, the dosage of about 240 mg/kg body weight per day may provide the effect of suppressing the hyperphagia sufficiently. Even if the dosage of the hydrolysate exceeds the dosage described above, there is little change in the effect of treating the hyperphagia. Therefore, the upper-limit of the dosage is preferably 320 mg/kg body weight per day or less.

The therapeutic agent according to the present invention is preferably prepared as a preparation so that the dosage of the active ingredient (casein hydrolysate according to the present invention) per day is within said range. Further, it is preferable to administer the same so that the dosage of the active ingredient (casein hydrolysate according to the present invention) per day is within said range.

The pharmaceutical preparation can be prepared, by known method, for example, using the active ingredient (casein hydrolysate according to the present invention) and optional additive(s) such as a pharmaceutically acceptable excipient. When the preparation is prepared, additive(s) such as excipient, binder, disintegrator, lubricant, stabilizer, flavor, diluent, solvent for injection and the like can be used.

In the case where the therapeutic agent according to the present invention consists of the composition comprising the active ingredient (casein hydrolysate) according to the present invention) and other ingredient(s) such as additive(s), the content of the active ingredient in said composition is usually 0.1 to 90 % by mass, preferably 0.5 to 40 % by mass, and more preferably 1 to 20 % by mass, though it is not particularly restrained (restricted).

As the excipient, for example, sugar and derivertives thereof, such as lactose, sucrose, glucose, mannitol, sorbitol; starch and derivertives thereof, such as corn starch, potatostarch, α-starch, dextrin, calboxymethylstarch; cellulose and derivertives thereof, such as crystalline cellulose, hydroxypropylcellulose, hydroxypropylmethyl cellulose, carboxymethyl cellulose, calcium carboxymethyl cellulose; gum arabic; dextran; pullulan; silicate and derivatives thereof such as light anhydrous silicic acid, synthetic aluminum silicate, magnesium aluminometasilicate; phosphate and derivatives thereof such as calcium phosphate; carbonate and derivatives thereof such as calcium carbonate; sulfate and derivatives thereof such as calcium sulfate) and the like may be mentioned.
As binder, for example, in addition to said excipient, gelatin; polyvinyl pyrrolidone; macrogol and the like can be mentioned.
As a disintegrator, for example, in addition to said excipient, chemically modified starch and cellulose derivatives such as sodium croscarmellose, sodium carboxymethylstarch, cross-linked polyvinyl pyrrolidone are mentioned.
As a lubricant, for example, talc; stearic acid; metal salts of stearic acid such as calcium stearate, magnesium stearate; colloidal silica; waxes such as Veegum, spermaceti; boric acid; glycol; carboxylic acid such as fumaric acid, adipic acid; sodium carboxylate such as sodium benzoate; sulfate such as sodium sulfate; leucine; lauryl sulfate such as sodium lauryl sulfate, magnesium lauryl sulfate; silicates such as silicic acid anhydride, silicate hydrate; starch derivatives and the like are mentioned.
As a stabilizer, for example, esters of parallydroxybenzoate such as methylparaben, propylparaben; alcohols such as chlorobutanol, benzyl alcohol, phenyl ethyl alcohol; benzalkonium chloride; acetic anhydride; sorbic acid and the like are mentioned.
As a flavor; for example, a sweetener, acidulant, spice(s) and the like are mentioned.
As solvent for injection, for example, water, ethanol, glycerol and the like are mentioned.

The therapeutic agent according to the present invention also can be orally administered, in addition to the use application as said medicament, after it is formulated in the food and drink, and feed (livestock feed). The food and drink containing the therapeutic agent according to the present invention includes the casein hydrolysate according to the present invention as an active ingredient, and thus, they have the effect for treating eating disorders, especially the hyperphagia. In a preferred embodiment, said food and drink can be preferably used for decreasing the blood glucose level of the patient with hyperphagia, accompanied with the effect of treatment of hyperphagia.

Such foods and drinks can be prepared by mixing sugars such as dextrin, starch; proteins such as gelatin, soy protein, corn protein; amino acids such as alanine, glutamine, isoleucine; polysaccharides such as cellulose, gum arabic; oils and fats such as soy oil, medium-chain triglyceride and the like into the casein hydrolysate.

As for the forms of foods and drinks, ones which can be routinely (daily) taken are preferable. For example, refreshing drinks, carbonated drinks, stamina drinks, fruit beverage, lactic acid drink and the like (including concentrate solution and instant powder of these drinks); frozen dessert such as ice cream, ice sherbet, ice shavings and the like; noodles such as soba, Japanese wheat noodle, strip of bean-jelly, coating of gyoza, coating of Chinese-style steamed meat, Chinese noodle, instant (Chinese) noodles; confectionery such as candy, chewing gum, chocolate, tablet confectionery, munchy, biscuit, jelly, jam, cream, baked goods; processed marine and stock farm products such as boiled (steamed) fish paste, ham, sausage and the like; dairy products such as processed milk, fermented milk; oils and fats and processed foods thereof such as salad oil, ternpura oil, margarine, mayonnaise, shortening, whipped cream, dressing; seasoning such as sauce, gravy; soup, stew, salad, daily dish, pickle, bread; enteral nutrition product; functional food and the like can be mentioned. In a preferred embodiment, these foods and drinks can be preferably used as a functional food used for treating the eating disorders of the patients with eating disorders, for treating the hyperphagia of the patients with hyperphagia, and for decreasing the blood glucose level of the patient with the hyperphagia.

The feed (livestock feed) containing the therapeutic agent according to the present invention includes the casein hydrolysate as an active agent, and thus, it has a therapeutic effect according to the present invention.
Such feed can be prepared by formulating, for example, into the casein hydrolysate, grains such as corn, wheat, barley, rye, milo; vegetable oilcake such as soybean cake and meal, rapeseed cake, coconut cake, linseed cake and the like; brans such as wheat bran, rice bran, defatted rice bran; manufactured dregs such as corn gluten meal, corn jam meal; animal-based feed such as fish meal, non-fat dry milk, whey, yellow grease, tallow; yeasts such as torula yeast, brewery yeast; mineral feed such as calcium carbonate; oils and fats; simple amino acid; saccharides and the like.
The forms of feed are preferably ones which can be routinely (daily) fed. For example, pet food, livestock feed, food for fishes and the like are mentioned.

While the therapeutic agent for eating disorders according to the present invention can be used alone, it also can be used together with other pharmaceutical composition, food and drink, or feed having the therapeutic effect for eating disorders. The therapeutic agent according to the present invention can increase the therapeutic effect for eating disorders by using together with these products. The pharmaceutical composition, food and drink, or feed used together with the therapeutic agent according to the present invention can be contained as an active ingredient in the pharmaceutical composition, food and drinks, or feed related to the present invention. Alternatively, they can be combined, as separate products, with the pharmaceutical composition, food and drink, or feed of the present invention without being contained in the latter for commercialization.

### [Electrophoresis]

Electrophoresis (two-dimension electrophoresis) in EXAMPLE can be performed as described below.

### (1) First dimension

First, the sample was dissolved in the swelling buffer (for example, Invitrogen Corporation) so that the concentration thereof would be 5 mg/ml, and then, a commercially available strip gel for isoelectric focusing (for example, Invitrogen Corporation) was swollen in the solution, and incubated at rest overnight. On said gel incubated at rest overnight, an electrophoresis is performed at 175 V for 20 minutes, and then, at voltage gradient of from 175 V to 2000 V for 45 minutes, and then, at 2000 V for 30 minutes. The strip gel after electrophoresis is stained by soaking in the staining solution (fluid).

### (2) Second dimension

The strip gel after the first electrophoresis is applied to the gel for second dimensional electrophoresis (Invitrogen Corporation), and then, electrophoresed at constant voltage of 200 V, for 35 minutes. After the electrophoresis, the gel is stained with a CBB staining solution (fluid) (Invitrogen Corporation).

### [TOF/MS (time of flight mass spectrometer)]

In the present specification, time-of-flight mass spectrometer is sometimes referred to as TOF/MS.
TOF/MS in EXAMPLE can be performed, for example, as described below.
After two-dimension electrophoresis was performed by said electrophoresis procedure, digestions (decompositions) of each peptide found as a spot within the gel are performed, and then, each peptide is identified with TOF/MS measuring instrument (Bruker Daltonics K.K.).

The present invention will be explained in more detail below, by indicating concrete examples. However, the present invention is not limited within said examples.

### EXAMPLE 1

### (Preparation method 1 of the casein hydrolysate according to the present invention)

### 1) Step of preparing and sterilizing casein solution

123 g of rennet casein (Fonterra Co-operative Group Limited, protein content 80%) was added into 2300 g of purified water, and swollen. To this, cone-hydrochloric acid (Wako Pure Chemical Industries, Ltd., concentration 36.5%) was added until pH reached 2.8, and then the temperature of 85°C was incubated (kept) for 10 minutes for sterilization.

### 2) Step of digesting casein solution

The sterilized casein solution was cooled to 42°C, and then, 20 mg of pepsin (SIGMA) was added, and then, digestion was performed for 2 hours.

### 3) Step of inactivating the enzyme

After digestion, the temperature increased to 85 °C again and maintained for 10 minutes, and then, pepsin was inactivated.

### 4) Step of centrifugation

After the inactivation of pepsin, the solution was centrifuged with a centrifuge (Hitachi, Ltd.) at 10,000 rpm (20,000 G) for 20 minutes, and then a supernatant fraction (soluble fraction) was separated from a precipitate fraction (insoluble fraction). The temperature of the solution was 10 °C.

### 5) Step of drying

After the centrifugation, the supernatant fraction was freeze-dried, and thus, 24 g of casein hydrolysate was obtained. On the other hand, the precipitate fraction after the centrifugation was freeze-dried, and thus, 92 g of dried powder of the precipitate fraction was obtained.

### EXAMPLE 2

### (Preparation method 2 of the casein hydrolysate according to the present invention)

### 1) Step of preparing a casein solution and sterilizing the same

10 g of rennet casein (Fonterra Co-operative Group Limited, protein content 80%) was added into 200 g of purified water, and thus, it was swollen. To it, conc. hydrochloric acid (Wako Pure Chemical Industries, Ltd., concentration 36.5%) was added, and thus, pH was adjusted to 2.8. Then, it was sterilized by maintaining at 85°C, for 10 minutes.

### 2) Step of digesting the casein solution

The sterilized casein solution was cooled to 42°C, and then 4.88 mg of pepsin (BIOPHEDEX) was added to the solution, and thus, the digestion was performed for 2 hours.

### 3) Step of inactivating the enzyme

After the digestion, the temperature was increased again to 85°C, for 10 minutes, and thus, pepsin was inactivated.

### 4) Step of separation

The solution in which the pepsin was inactivated was passed through MF membrane (Asahi Kasei Corporation), and thus, separated into a permeate (soluble fraction) and a concentrate (insoluble fraction).

### 5) Step of drying

The permeate was freeze-dried, and thus, 2.2 g of the casein hydrolysate was obtained.

### EXAMPLE 3

### (Preparation of a therapeutic agent for hyperphagia in a form of tablet)

To 150 g of the casein hydrolysate obtained by the method of EXAMPLE 1, 100 g of lactulose powder (Morinaga Milk Industry Co., Ltd.), 635 g of maltodextrin (Matsutani Chemical Industry Co., Ltd.), 85 g of nonfat dry milk (skim milk) (Morinaga Milk Industry Co., Ltd.), 1 g of Stevia sweetening (San-Ei Gen F. F. I., Inc.), 5 g of yoghurt flavor (San-Ei Gen F. F. I., Inc.), and 24 g of a preparation of glycerol ester of fatty acids (Riken Vitamin Co., Ltd.) were added and mixed homogenously. Then, 1800 tablets (about 900 g) containing the casein hydrolysate (a therapeutic agent for hyperphagia), one tablet being 0.5 g, were prepared by tableting continuously said mixed powder, at rate of 12 tablets per minute, at pressure of 9.8 KPa, using a tableting machine (Hata Iron Works Co., Ltd.). The casein hydrolysate per one tablet was about 15% by mass.

### EXAMPLE 4

### (Preparation of enteral nutrient powder containing a therapeutic agent for hyperphagia)

10 kg of hydrolysate of whey protein (Morinaga Milk Industry Co., Ltd.), 36 kg of dextrin (Showa Sangyo Co., Ltd.), and a small amount of water-soluble vitamins and minerals were dissolved in 200 kg of water, and then, an aqueous phase was prepared in a tank. Further, 3 kg of soybean oil (Taiyo Yushi Corp.), 8.5 kg of palm oil (Taiyo Yushi Corp.), 2.5 kg of safflower oil (Taiyo Yushi Corp.), 0.2 kg of lecitin (Ajinomoto Co., Inc.), 0.2 kg of monoglyceride of fatty acids (Kao Corporation), and a small amount of fat-soluble vitamins were mixed and dissolved, and thus, an oily phase was prepared. To the aqueous phase in thank, the oily phase was added and mixed by stirring, and then, heated to 70°C, and then, further homogenized with a homogenizer at pressure of 1.4.7 MPa. Subsequently, the homogenate was sterilized at 90°C, for 10 minutes, and then, condensed, and by spray drying, about 59 kg of intermediate product powder was prepared. To 50 kg of the intermediate product powder, 6.8 kg of sucrose (Hokuren), 167 g of amino acids mixed powder (Ajinomoto Co., Inc.), and 1 kg of the casein hydrolysate obtained by the method of EXAMPLE 1 were added and mixed homogeneously, and thus, about 56 kg of enteral nutrient powder containing the casein hydrolysate was prepared.

### [Test example 1]

The object of this test was to identify the casein hydrolysate of EXAMPLE 1 which is the product according to the present invention.

### [Method of test]

### (1) Preparation of the sample

The casein hydrolysate according to the present invention, obtained by freeze-drying the supernatant fraction after centrifugation in EXAMPLE 1, (Sample 1, hereafter, also called as the product according to the present invention), the dried powder of the precipitate fraction, obtained by freeze-drying the precipitate fraction after centrifugation in EXAMPLE 1, (Sample 2, hereafter, also called as the precipitate fraction), and the rennet casein, as control, used in EXAMPLE 1 as casein (Sample 3, Fonterra Co-operative Group Limited) were used as samples.

### (2) Electrophoresis

### (First dimension)

Said samples were dissolved in the swelling buffer solution (Invitrogen) so that each concentration would be 5 mg/ml, and a commercially available strip gel for isoelectric focusing (Invitrogen) was swollen in the solution and incubated at rest overnight. These gels, incubated at rest overnight, were electrophoresed at 175 V for 20 minutes, at voltage gradient of from 175 V to 2000 V for 45 minutes, and further, at 2000 V for 30 minutes. The strip gels after electrophoresis were stained by soaking the same in the staining solution.

### (Second dimension)

The strip gel after the electrophoresis in the first dimension was applied to the gel for the second dimension (Invitrogen), and then, electrophoresed at constant voltage of 200 V, for 35 minutes. After the electrophoresis was completed, the gel was stained with CCB staining solution (Invitrogen).

### (3) Identification of the spots of the supernatant fraction

After the electrophoresis in the second dimension, each peptide recognized as a spot was digested in the gel, and each peptide was identified using TOF/MS measuring instrument (Bruker Daltonics K.K.).

### [Result]

In Sample (the product according to the present invention), molecules having a molecular weight more than 20 kD were almost disappeared. Further, in Sample 2 (the precipitate fraction), high-molecular weight compounds, which was detected in Sample 3 (rennet casein) and considered to be derived from the casein micelle and polymer (multimer) were almost disappeared. However, in Sample 2, a casein fraction detected at molecular weight of 10 kDa to 30 kDa remained as protein. The result of the molecular weight distribution is shown in Table 1. Numerical values described in the Table mean the percentage of the whole of each fraction.

Further, the analysis using TOF/MS was compared with a commercially available casein fraction samples. From this comparison, it was revealed that Sample 1 casein hydrolysate (the product according to the present invention) comprises about 80% of hydrolysate derived from αs2-casein and, partially, hydrolysate derived from αs1-casein or proteins and the like. The proportion of hydrolysate derived from αs2-casein was obtained by scanning the result of TOF/MS analysis and using the area analysis.
Molecular weight of αs2-casein has been known to be 25 kDa (see "Properties of ingredients of cow's milk and health", Kunio Yamauchi et al, Kouseikan Co., Ltd., June 1, 1993, First Edition, First Print, page 8). Therefore, it was revealed that the active ingredient of the hydrolysate has a molecular weight of no more than 25 kDa.
Further, from the result, it was revealed that in the molecular weight distribution of the product according to the present invention, 50% or more of ingredients are 1 kDa to 20 kDa.
On the other hand, it was revealed that the casein hydrolysate derived from β-casein and αs1-casein accounts for the greater part of the precipitate fraction.
In addition to the fraction obtained by centrifugation at 20,000 G for 20 minutes (Sample 1), the fraction obtained by centrifugation at 15,000 G for 60 minutes was analyzed in the same way. It was revealed that the fraction obtained by the centrifugation at 15,000 G for 60 minutes comprises the molecule having about the same molecular weight as said Sample 1.

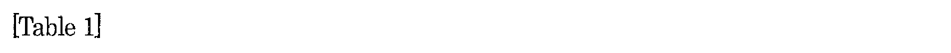

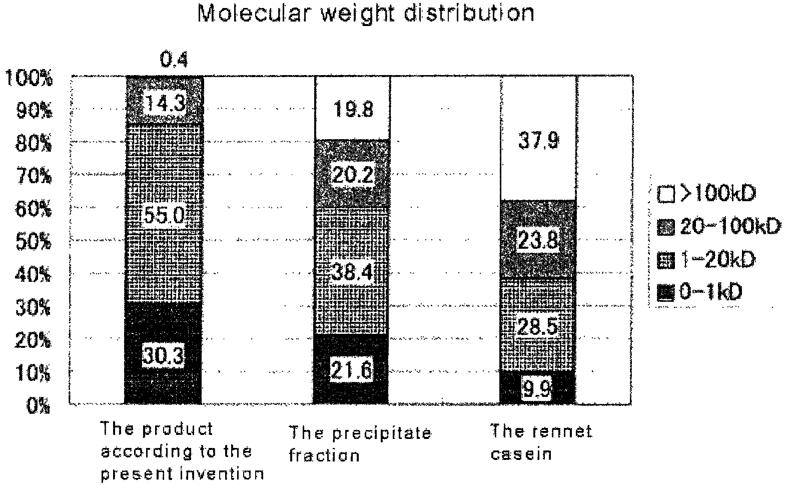

### [Test example 2]

The object of this test was to perform the amino acid analysis of the casein hydrolysate according to the present invention.

### [Method of test]

The casein hydrolysate of the present invention obtained in Test example 1 (Sample 1) and the precipitate fraction (Sample 2) were used. Each Sample was hydrolyzed with hydrochloric acid, and then, labeled with dansyl chloride (Sigma). This preparation was separated using C18 column (Waters). The peak obtained was detected with a fluorescence detector (Hitachi, Ltd.) and analyzed.

### [Result]

Both of the casein hydrolysate in EXAMPLE 1 (Sample 1) and the precipitate fraction (Sample 2) had the same amino acid composition as the rennet casein which is a raw material.

### [Test example 3]

The object of this test was to confirm that the casein hydrolysate according to the present invention has a therapeutic effect against the hyperphagia.

### (1) Preparation of sample

Control: A distilled water for injection was used as it is.
The product according to the present invention: The casein hydrolysate according to the present invention, prepared by the method described in EXAMPLE 1, was diluted with the distilled water so that the concentration of the hydrolysate would be 5 mg/ml.
The precipitate fraction: Sample 2 in the Test Example 1 was diluted with the distilled water into 5 mg/ml.
Whole casein hydrolysate: The rennet casein (Fonterra Co-operative Group Limited, protein content 80%) used in EXAMPLE 1 was digested with pepsin in the same way as in EXAMPLE 1, and then, without centrifuging, diluted with the distilled water for injection so that the concentration of the hydrolysate would be 5 mg/ml.
GMP (glycol-macropeptide): A commercially available GMP (MG Nutritionals) was diluted with the distilled water for injection so that the concentration of GMP would be 5 mg/ml.
αs-casein: A commercially available αs-casein (Sigma) was diluted with the distilled water for injection so that the concentration of αs-casein would be 5 mg/ml.
κ-casein: A commercially available κ-casein (Sigma) was diluted with the distilled water for injection so that the concentration of κ-casein would be 5 mg/ml.
Whole casein: A commercially available lactic acid casein (Fonterra Co-operative Group Limited) was diluted with the distilled water for injection so that the concentration of said casein would be 5 mg/ml.

### (2) Feed (livestock feed)

A commercially available livestock feed containing no protein from milk (Nihon Nosan, trade name: MR Stock) was used. As drinking water; tap water (city water) was used.

### (3) Test animal

As animal model for hyperphagia, KK-Ay mice (male, 5 weeks old, purchased from CLEA Japan, Inc.) were used.

### (4) Method of test

After pre-feeding with said livestock feed for 10 days, test animals were divided into 8 groups each including 8 animals so that they would have the nearly equal body weight and take the nearly equal amount of livestock feed. The animals of each group could take the livestock feed and water freely. During 5 days of one week, 0.5 ml of the sample was administered to each animal per day using a sonde. The test was performed for 5 weeks. The amount of livestock feed taken by the animal was determined once a week.
Each group is as described below.
Group 1 (Control)
Group 2 (The product according to the present invention)
Group 3 (The precipitate fraction)
Group 4 (Whole casein hydrolysate)
Group 5 (GMP)
Group 6 (αs-casein)
Group 7 (κ-casein)
Group 8 (Whole casein)

### (5) Result of the test

Relative amount of the livestock feed taken by each group 5 weeks after the starting of administration is shown in Table 2.
Relative amount of the livestock feed taken is an average amount of the livestock feed taken by each group when the average amount of livestock feed taken by control group is considered 1. With the product according to the present invention, the amount of livestock feed taken by each group decreased significantly compared to the control. On the other hand, as for the precipitate fraction, though a tendency of increase of the amount of the livestock feed taken by the animal was observed, it wasn't a significant difference. Further, as for GMP derived from κ-casein, though a tendency of decrease of the amount of the livestock feed taken by the animal was observed, it wasn't a significant effect compared to the control. Further, as for κ-casein, though a tendency of decrease of the amount of the livestock feed taken by the animal was observed, it wasn't a significant effect. As for αs-casein, though a tendency of increase of the amount of the livestock feed taken by the animal was observed, it wasn't a significant effect. As for whole casein, though a tendency of increase of the amount of the livestock feed taken by the animal was observed, it wasn't a significant effect. As for whole casein hydrolysate, though a tendency of decrease of the amount of the livestock feed taken by the animal was observed, it wasn't a significant effect. Thus, it was revealed that when whole casein was hydrolyzed and a supernatant fraction (water-soluble fraction) was separated by removing the precipitate fraction, a significant effect of treating the hyperphagia was generated.
From these results, it was revealed that the product according to the present invention (Group 2) has a therapeutic effect against the hyperphagia. Further, it was revealed that the casein hydrolysate according to the present invention has a remarkable effect for suppressing the hyperphagia, in comparison with other casein fractions and whole casein hydrolysate.

**[Table 2]**

| Group | Test sample | Relative amount of livestock feed ingested 5 weeks later |
|---|---|---|
| 1 | Control | 1.000 |
| 2 | The product according to the present invention | 0.885* |
| 3 | The precipitate fraction | 1.019 |
| 4 | Whole casein hydrolysate | 0.979 |
| 5 | GMP | 0.997 |
| 6 | αs-casein | 1.022 |
| 7 | κ-casein | 0.990 |
| 8 | Whole casein | 1.087 |

| | | |
|---|---|---|
| *significant, provided that p<0.05 | | |

### [Test example 4]

The object of this test was to confirm that the casein hydrolysate according to the present invention has an effect of decreasing the blood glucose level of the patient with hyperphagia.

### (1) Preparation of sample

The same one as in EXAMPLE 3 was used.

### (2) Feed (Livestock feed)

A commercially available livestock feed containing no protein from milk (Nihon Nosan, trade name: MR Stock) was used. As drinking water, tap water (city water) was used.

### (3) Test animal

As animal model for hyperphagia, KK-Ay mice (male, 5 weeks old, CLEA Japan, Inc.) were used.

### (4) Method of test

After pre-feeding with said livestock feed for 10 days, test animals were divided into 8 groups each including 8 animals so that they would have the nearly equal body weight and take the nearly equal amount of livestock feed. The animals of each group could take the livestock feed and water freely. During 5 days of one week, 0.5 ml of the sample was administered to each animal per day using a sonde. The test was performed for 5 weeks. The blood glucose level was determined once a week, using a glucose kit for self checking (Johnson & Johnson).
Sample of each group is as described below.
Group 1 (Control)
Group 2 (The product according to the present invention)
Group 3 (The precipitate fraction)
Group 4 (Whole casein hydrolysate)
Group 5 (GMP)
Group 6 (αs-casein)
Group 7 (κ-casein)
Group 8 (Whole casein)

### (5) Result of the test

Relative blood glucose levels 5 weeks after the starting of administration are shown in Table 3. Relative blood glucose level is an average blood glucose level of each group when the average blood glucose level of the control group is considered 1. With the product according to the present invention, the blood glucose levels decreased significantly compared to the control. With whole casein hydrolysate, whole casein and κ-casein, though a tendency of decrease of the blood glucose levels was observed, it wasn't a significant effect. Further, with the precipitate fraction, GMP and αs-casein, though a tendency of increase of the blood glucose level was observed, it wasn't a significant effect.

### (6) Correlation between these effects and the effect of treatment of hyperphagia

Further, the correlation between the blood glucose level decreasing effect and the effect of treatment of hyperphagia was tested. Pearson's coefficient of correlation was 0.593, which was significant at p<0.01.

**[Table 3]**

| Group | Test sample | Relative amount of livestock feed ingested 5 weeks later |
|---|---|---|
| 1 | Control | 1.000 |
| 2 | The product according to the present invention | 0.716* |
| 3 | The precipitate fraction | 1.038 |
| 4 | Whole casein hydrolysate | 0.854 |
| 5 | GMP | 1.171 |
| 6 | αs-casein | 1.107 |
| 7 | κ-casein | 0.934 |
| 8 | Whole casein | 0.760 |

| | | |
|---|---|---|
| * Significant difference was observed, provided that p<0.05 | | |

### [Test example 5]

The object of this test was to confirm the effect of the casein hydrolysate according to the present invention on the appetite and blood glucose level when the hydrolysate was administered to the normal animals with no symptom of hyperphagia.

### (1) Preparation of sample

Control: A distilled water for injection was used as it is.

The product according to the present invention: The casein hydrolysate prepared by the method described in EXAMPLE 1 was diluted with the distilled water so that the concentration of the hydrolysate would be 5 mg/ml.

### (2) Feed (Livestock feed)

A commercially available livestock feed containing no protein from milk (Nihon Nosan, trade name: MR Stock) was used. As drinking water, tap water (city water) was used.

### (3) Test animal

KK mice (male, 5 weeks old, CLEA Japan, Inc.) which are control of KK-Ay mice were used as animal model with no symptom of hyperphagia.

### (4) Method of test

After pre-feeding with said livestock feed for 10 days, test animals were divided into 2 groups each including 8 animals so that they would have the nearly equal body weight and take the nearly equal amount of livestock feed. The animals of each group could take the livestock feed and water freely. During 5 days of one week, 0.5 ml of the sample was administered to each animal per day using a sonde. The test was performed for 5 weeks. During the test period, the amount of the livestock feed taken by the animals and the blood glucose level were determined once a week. For determining the blood glucose level, a glucose kit for self checking (Johnson & Johnson) was used.

### (5) Result of the test

The results of relative amount of livestock feed taken by the animals and relative blood glucose level 5 weeks after the starting of the administration are shown in Table 4. Though t-test of Student was performed, the effect of the product according to the present invention for suppressing the amount, of the livestock feed taken by KK mice and increase of blood glucose level of the animals cannot be observed. Namely, it was revealed that the product according to the present invention has no influence on the normal animals having no symptom of hyperphagia. From this fact, it is indicated that the product according to the present invention does not suppress the ordinary appetite appropriately expressed (normal appetite) and can be safely taken (ingested).

**[Table 4]**

| Test sample | Relative amount of the livestock feed ingested | Relative blood glucose level |
|---|---|---|
| Control | 1.000 | 1.000 |
| The product according to the present invention | 0.982 | 1.016 |

### [Test example 6]

The object of this test is to specify the amount based on the normal appetite and the amount based on the excessive appetite among the amount of the livestock feed taken by the animal with the symptom of hyperphagia (hyperphagia animal).

### (1) Feed (Livestock feed)

A commercially available livestock feed containing no protein from milk (Nihon Nosan, MR Stock) was used. As drinking water, tap water (city water) was used.

### (2) Test animal

As animal model for hyperphagia, KK-Ay mice (male, 5 weeks old, purchased from CLEA Japan, Inc.) were used. KK mice (male, 5 weeks old, purchased from CLEA Japan, Inc.) which are control of KK-Ay mice were used as an animal model with no symptom of hyperphagia. Further, as animal model for hyperphagia, ZDF rats (male, homo, 5 weeks old, purchased from Charlesriver Laboratories Japan, Inc.) were used. Zucker lean rats (male, 5 weeks old, purchased from Charlesriver Laboratories Japan, Inc.) which are control of ZDF rats were used as an animal model with no symptom of hyperphagia.

### (Test animal)

### Mice

(i) KK mice which are controls of KK-Ay mice (hereinafter, also referred to as control 1)
(ii) KK-Ay mice
   Rats
(iii) Zucker lean rats which are controls of ZDF rats (hehrinafter, also referred to as control 2)
(iv) ZDF rats

### (3) Method of test

Said mice and rats were allowed to take the livestock feed and water freely.
As for mice, the amounts of the livestock feeds taken by the animals were compared after the acclimation period for 10 days after the arrival thereof. As for rats, the amounts of the livestock feed taken by the animals were compared after the feeding for 13 weeks after the arrival thereof, because substantial time is required for ZDF rats to exhibits the symptom of hyperphagia.

### (4) Result

The result of the average amount of the livestock feed taken by the animals after 5 weeks was shown in Table 5. Control 1 means KK mice, and control 2 means Zucker lean rats. KK-Ay mice took 1.22 times livestock feed compared to control, and ZDF rats took 1.23 times livestock feed compared to control. Namely, with regard to both of the mice and rats with hyperphagia, it was revealed that the amount of livestock feed taken by the animals with hyperphagia increased by about 20% in comparison with that of the control normal animals. From this fact, the excessive appetite associated with hyperphagia can be considered to be about 20% compared with the amount of the livestock feed taken due to the normal appetite.

**[Table 5]**

| Test sample | Average amount of livestock feed ingested (g/day) | Increasing rate |
|---|---|---|
| Normal mouse | 5.26 | 1.00 |
| Hyperphagia mouse | 6.43 | 1.22 |
| Normal rat | 26.2 | 1.00 |
| Hyperphagia rat | 32.2 | 1.23 |

| | | |
|---|---|---|
| Notes: The rate of KK-Ay mice is based on the amount of livestock feed of Control as standard. The rate of ZDF rats is based on the amount of livestock feed of Control 2 as standard. | | |

### [Test example 7]

The object of this test was to confirm that the effect of the therapeutic agent according to the present invention is dose-dependent.

### (1) Preparation of sample

### Control: Distilled water for injection was used.

High dosage sample: The casein hydrolysate in EXAMPLE 1 was diluted with distilled water so that the concentration of the hydrolysate would be 5 mg/ml.
Low dosage sample: The casein hydrolysate prepared in EXAMPLE 1 was diluted with distilled water so that the concentration of the hydrolysate would be 0.5 mg/ml.

### (2) Feed (Livestock feed)

A commercially available livestock feed containing no protein from milk (Nihon Nosan, MR Stock) was used. As drinking water, tap water (city water) was used.

### (3) Test animal

As animal model for hyperphagia, KK-Ay mice (male, 5 weeks old, CLEA Japan, Inc.) were used.

### (4) Method of test

0.5 ml of said sample was administered to each animal once per day in 5 days per week. The period for administration was 5 weeks, during which the animals could take the livestock feed and water freely.

### (5) The method for estimate

The amount of the livestock feed taken by KK-Ay mice and the blood glucose level thereof were determined once a week.
For determining the blood glucose level once a week, a glucose kit for self checking (Johnson & Johnson) was used.

### (6) Result

Relative amount of the livestock feed taken by the animals of each group and blood glucose levels thereof 5 weeks after the starting of administration are shown in Table 6. When the high dosage sample was used, significant suppression of the amount of the livestock feed taken by the animals and suppression of increase of the blood glucose level were observed. On the other hand, using the low dosage sample, no significant effect was observed.
Accordingly, it was revealed that the therapeutic agent for hyperphagia according to the present invention has a dose-dependency.
The dosage in the group of the high dosage sample was 2.5 mg (5 mg/ml X 0.5 ml) per day. The average body weight of KK-Ay mice to which the samples were administered was about 43 g.
Accordingly, it was revealed that said effect would be obtained when 290 mg/kg body weight or more of the casein hydrolysate according to the present invention are taken once a week.

**[Table 6]**

| Test sample | Relative amount of the livestock feed ingested | Relative blood glucose level |
|---|---|---|
| Control | 1.000 | 1.000 |
| Low dosage sample | 0.985 | 1.070 |
| High dosage sample | 0.885* | 0.716* |

| | | |
|---|---|---|
| Significant difference was observed, provided that p<0.05 | | |

### [Test example 8]

The object of this test was to study the period from taking the casein hydrolysate according to the present invention to expressing the effect thereof, and the period (duration) of said effect.

### (1) Preparation of sample

### Control: a distilled water for injection was used.

The product according to the present invention: The casein hydrolysate prepared in EXAMPLE 1 was diluted with the distilled water for injection so that the concentration of the hydrolysate would be 5 mg/ml.

### (2) Test animal

As the animal model for hyperphagia, ZDF rats (male, 5 weeks old, purchased from Charles river Laboratories Japan, Inc.) were used. ZDF rats were pre-fed for 12 weeks in order to develop the hyperphagia.

### (3) Livestock feed

A commercially available livestock feed containing no protein from milk (Nihon Nosan, MR Stock) was used. As drinking water, tap water (city water) was used.

### (4) Method of test

After the following observation period, the livestock feed was administered.

### [Observation period. 1]

The amount of the livestock feed taken by the animals was observed for one week, with no particular treatment.

### [Observation period 2]

At the starting, the amount of the livestock feed taken by the animals was determined, and then, the distilled water for injection was administered, and then, the amount of the livestock feed taken by the animals was observed for one week. Thus, it was confirmed that no change of the pattern of taking the livestock feed by administration of the distilled water for injection using sonde was observed in comparison to the rhythm of taking the livestock feed during the observation period 1. After the observation period 2, the body weight and blood glucose level of the ZDF rats were determined, and the ZDF rats were divided into a group for control and a group to be administered with the product according to the present invention so that total of the body weights and the amount of livestock feed taken by the animals of these two groups would be nearly equal. Each group included 8 ZDF rats.

### [Administration]

After the observation period 2, the group to be administered with the product according to the present invention was administered, with 2 ml of the sample prepared with the product according to the present invention, once per day in 5 days per week, and in the same way, the control group was administered with the distilled water for injection, and then, the amount of the livestock feed taken by them was observed for 2 weeks.
During the test period, the amount of the livestock feed taken by the animals was determined 5 times per week.

### (5) Result

The observed values of the amount of the livestock taken by the animals during the observation periods are shown in Figure 1. In the Figure 1, the amounts of the sample administered and the amounts of the livestock feed taken by the animals were not determined on Saturday and Sunday, and therefore, the data on these days were omitted from Measurement date. Water for injection indicates the distilled water for injection.
"Sample" between the observation period 2 and 3 indicates that the product according to the present invention or the distilled water for injection was administered to each group.
During the observation period 1, it was confirmed that the daily change of the amount of livestock feed taken by ZDF rats is about ±2 g. During the observation period 2, it was confirmed that administration of the distilled water of injection does not affect the pattern of taking the livestock feed, and therefore, that the stress by administration using the sonde does not affect the pattern.
From 4 days after the starting the administration of the casein hydrolysate according to the present invention, a significant decrease of the amount of the livestock feed taken by the group of the product of the present invention in comparison to the control group. Surprisingly, this effect continued to 9 days after the starting the administration.
From this result, it was revealed that the suppressing effect on the amount of the livestock feed taken by the animals by the casein hydrolysate according to the present invention is not immediate one, the effect is exerted about 4 days after the administration, and the effect continues for about 6 days.
This result indicates that the present product according to the present invention does not immediately induce feeling of fullness (plenitude). Therefore, the timing of taking the product according to the present invention is not limited before and/or after the meal, or between the meals. Further, it is not required to take every day because the effect of suppressing the hyperphagia is exerted for a certain period.

### [Test example 9]

The object of this test was to specify the molecular weight of the active ingredient of the casein hydrolysate according to the present invention.

### (1) Preparation of sample

The following samples were prepared.

### Control: the distilled water for injection was used. (Control)

The product of the present invention: The casein hydrolysate prepared in EXAMPLE 1 (the product according to the present invention) was diluted with the distilled water for injection so that the concentration of the hydrolysate would be 5 mg/ml.
Re-digested sample: To 33 g of the casein hydrolysate prepared in EXAMPLE 1 (the product according to the present invention), 66 mg of pepsin was added, and digestion was performed at pH 2.8 and at 42°C, for 2 hours, and then, lyophilization (freeze-drying) was performed. This lyophilized (freeze-dried) preparation was dissolved with the distilled water for injection so that the concentration of the hydrolysate would be 5 mg/ml.
It was confirmed that the re-digested sample has a molecular weight less than 1 KDa.

### (2) Test animal

As animal model for hyperphagia, KK-Ay mice (male, 5 weeks old, purchased from CLEA Japan, Inc.) were used.

### (3) Feed (Livestock feed)

A commercially available livestock feed containing no protein from milk (Nihon Nosan, MR Stock) was used. As drinking water, tap water (city water) was used.

### (4) Method of test

The animals were divided into three groups, each group including 8 animals, so that they would have the nearly equal body weight and take the nearly equal amount of livestock feed. During 5 days of one week, 0.5 ml of the sample was administered to each animal per day using a sonde.
The dosing period was 5 weeks, and the animals of each group could take the livestock feed and drinking water freely.

### (5) Result

Relative amounts of the livestock feed taken by each group were compared 5 weeks after the starting of administration. As for the group to which the product according to the present invention was administered, the amount of livestock feed taken by the group was significantly decreased in comparison to that of the control.
On the other hand, as for the group to which the re-digested sample was administered, the amount of livestock feed taken by the group did not show a difference from that of the control, and thus, any significant effect could not observed. From the fact that the re-digested sample is an ingredient having a molecular weight of no more than 1 kDa, it was revealed that the active ingredient of the therapeutic agent for hyperphagia according to the present invention is the ingredient having a molecular weight of 1 kDa or more.
Consequently, it was revealed that the ingredient which has the effect of the present invention for suppressing the hyperphagia is in the range of molecular weight of from 1 kDa to 37 kDa. It was revealed, considering together this result and the result of Test example 1, that the ingredient which has the effect of the present invention for suppressing the hyperphagia is in the range of molecular weight of from 1 kDa to 25 kDa.

### INDUSTRIAL APPLICABILITY

According to the present invention, the therapeutic agent for eating disorders, especially hyperphagia, which has a high safety to a human being and animals, and thus, can be routinely (daily) administered or taken, can be obtained. The therapeutic agent according to the present invention also can be used as an agent for decreasing the blood glucose level of the patient affected with the hyperphagia.
Further, the casein hydrolysate which is an active ingredient in the therapeutic agent according to the present invention can be produced in large scale from the raw materials such as milk, and therefore can be provided at a low price.

## Claims

1. (Amended) A therapeutic agent for eating disorders comprises, as an active ingredient, a fraction of peptide fragment having a molecular weight of 1 kDa to 25 kDa, wherein the peptide fragment comprises αs2-casein hydrolysate by pepsin.

2. l(Canceled)

3. (Amended) The therapeutic agent for eating disorders according to claim 1, wherein the fraction of peptide fragment having a molecular weight of 1 kDa to 25 kDa is a water-soluble fraction of hydrolysate obtained by hydrolyzing 1 mass part of casein with 1/10,000 to 1/1,000 mass part of pepsin.

4. (Amended) The therapeutic agent for eating disorders according to claim 3, wherein the water-soluble fraction is a supernatant fraction obtained by fractionating the casein hydrolysate by pepsin by decantation or centrifugation.

5. (Amended) The therapeutic agent for eating disorders according to any of claims 1, 3 and 4, wherein the amount of ingested meal per day in hyperphagia is decreased at least 10% after 5 weeks from the starting of administration, in comparison to the starting of administration.

6. (Amended) The therapeutic agent for eating disorders according to any of claims 1 and 3 to 5, wherein the eating disorder is hyperphagia.

7. (Amended) A method for preparing an therapeutic agent for eating disorders, comprising steps of:
obtaining casein hydrolysate by pepsin by hydrolyzing casein with pepsin by adding 1/10000 to 1/1000 mass parts of pepsin to 1 mass part of casein,
obtaining a supernatant fraction, as a water-soluble fraction of peptide fragment having a molecular weight of 1kDa to 25 kDa, wherein the peptide fragment comprises αs2-casein hydrolysate by pepsin, by fractionating the casein hydrolysate by pepsin by decantation or centrifugation.

8. (Canceled)

9. (Canceled)

10. (Amended) The method according to claim 7, wherein the eating disorder is hyperphagia.

11. (Canceled)

12. (Amended) A method for treating eating disorders by administering the therapeutic agent for eating disorders according to any of claims 1 and 3 to 6 to a patient with eating disorder with anxiety of transition to anorexia.

13. (Amended) Use of a fraction of peptide fragment having a molecular weight of 1kDa to 25 kDa, wherein the peptide fragment comprises αs2-casein hydrolysate by pepsin, in the manufacture of the therapeutic agent for eating disorders according to any of claims 1 and 3 to 6.

14. (Amended) A fraction of peptide fragment having a molecular weight of 1 kDa to 25 kDa, wherein the peptide fragment comprises αs2-casein hydrolysate by pepsin, for use in treating eating disorders.
